# EUROPEAN PATENT APPLICATION

(11) **EP 1 785 135 A1**
(43) Date of publication of application: **16.05.2007**
(21) Application number: 05380248.4
(22) Date of filing: 10.11.2005
(51) Int. Cl.: A61K 31/4439, A61K 9/50

(54) **New stabilized galenic formulations comprising lansoprazole and their preparation**

(71) Applicant: LABORATORIOS DEL DR. ESTEVE, S.A., 08041 Barcelona (ES)
(72) Inventor: Lopez Cabrera, Antonio, 08041 Barcelona (ES); Lizcano Garcia, Xavier, 08041 Barcelona (ES)
(74) Representative: ABG Patentes, S.L.

(57) **Abstract**

New stable oral pharmaceutical preparation containing lansoprazole or a salt thereof as active ingredient, comprising:
a) a nucleus containing an inert core coated with a first layer comprising Lansoprazole or salts thereof, optionally mixed with an alkaline reacting compound, and an inert water soluble polymer,
b) an inert coating disposed on said nucleus, comprising a water soluble polymer; and
c) an enteric outer layer disposed on the previous coating comprising a gastric resistant polymer;

wherein the preparation is characterized in that no anti-tacking agent is present in the first layer or in the inert coating.

## Description

### Field of the invention

The present invention is related to new stable pharmaceutical preparations for oral administration containing lansoprazole or salts thereof, optionally mixed with an alkaline reacting compound, characterized in that the formulations do not contain any anti-tacking agent, like for example talc. The invention also relates to a method for the manufacture of such preparations.

### Background of the invention

Lansoprazole is an acid labile benzimidazole derivative very effective for the treatment of gastric and duodenal ulcers, gastroesophageal reflux disease, severe erosive esophagitis, Zollinger-Ellison syndrome and H-pylori eradication. However, it is well known that this compound has poor stability. In the solid state it is susceptible to heat, moisture and light, and in aqueous solution or suspension its stability decreases with decreasing pH. The degradation of lansoprazole is catalyzed by acidic reacting compounds, i.e. acidic compounds or compounds which react like acidic compounds.

Pharmaceutical preparations containing acid labile compounds have to be subcoated in order to avoid a reaction between the active ingredient and the outer acidic enteric coating which reaction -if occurring- would result in degradation, destabilization and consequently discolouration of the active ingredient.

The use of a barrier layer to protect the pharmaceutical from degradation caused by an enteric coating is well known from the prior art. Nevertheless, it is not possible to use conventional enteric coatings in a conventional way for acid labile benzimidazole compounds since decomposition takes place and the preparations become discoloured and lose the active ingredient content with time. Prior art partially avoids the above mentioned stability problem by including an alkaline salt form of the benzimidazole compound or incorporating an alkaline reacting compound (i.e. a base or compounds that react like bases, i.e. magnesium oxide, hydroxide or carbonate, aluminium hydroxide, aluminium, calcium, sodium or potassium carbonate, phosphate or citrate, composite aluminium/magnesium compounds, alkaline aminoacids, N-methyl-D-glucamine, etc.) into an enteric coated preparation. As described in US-A-4,786,505, US-A-5,232,706, EP-A-237200, EP-A-124495, US-A-5,385,739, EP-A-519144, the alkaline reacting compound may be present within or on the surface of the nucleus together with the benzimidazole compound. Some authors use the alkaline reacting compound also in the composition of an isolation layer to ensure stability of these forms. It is important to note that patent US-A-4,786,505, in its Example 1, Table 1 No. 1, illustrates a formulation which is free of such alkaline reacting compound, and that according to Table 3 (No. 1-II) this formulation has a rather poor stability.

EP 993 830 B1, refers to a high stability solid pharmaceutical enteric coated composition of an acid labile benzimidazole.

The composition claims:
a) a nucleus formed by an inert core coated with a first layer consisting of the acid labile benzimidazole compound, an inert water soluble polymer selected from hydroxypropylmethylcellulose and hydroxypropylcellulose and talc;
b) an inert coating disposed on said nucleus, formed by a water soluble polymer selected from hydroxypropylmethylcellulose and hydroxypropylcellulose, talc and a pigment; and
c) an outer layer disposed on the previous coating comprising an enteric coating consisting of a gastric resistant polymer such as co-polymerized methacrylic acid/methacrylic acid methyl esters, a plasticizer such as triethylcitrate and talc.

Thus, according to the state of the art, in enteric coated pharmaceutical compositions containing an acid labile benzimidazole compound, either the association of an alkaline substance with the active ingredient or the use of a very particular combination of excipients are recommended in order to ensure the stability of the drug for long term storage. Further, it is also recommended in the state of the art the presence of anti-tacking agents, like talc to facilitate the production process of the referred compositions.

### Outline of the invention

According to the present invention, high stability enteric coated solid preparations containing lansoprazole or salts thereof, optionally mixed with an alkaline reacting compound, are obtained. The enteric coated new galenic formulations do not contain any anti-taking agent in the nucleus, formed by a first active layer coated on an inert core or in the neutral coating disposed on said nucleus.

The aqueous polymeric dispersions usually exhibit a high degree of "tack" (stickiness) on spraying which leads to aggregation during coating. Effective anti-tacking agents in use are normally talc or fumed silica which facilitate the production process. It was surprisingly found in the present invention that not adding any anti-tacking agent to the polymeric dispersions constituting the active layer or to the inert intermediate layer did not lead to stacking effects and that neither of both formulations, with or without anti-tacking agent, affects the production process. Both formulations exhibit the same stability profile under accelerated conditions.

The new preparation is characterized in that to an inert sugar/starch spherical core, a first layer is applied comprising a mixture of lansoprazole as active ingredient or a salt thereof, optionally mixed with an alkaline reacting compound, an inert water soluble polymer and, optionally, pharmaceutical acceptable excipients with the exclusion of any anti-tacking agent, followed by a second layer comprising an inert water soluble polymer and compatible excipients, with the exclusion of any anti-tacking agent. Finally, a third layer comprising a gastric resistant polymer is applied. The core, the process conditions and the excipients have been selected in order to obtain the required coating efficiency for each layer.

The resulting new preparation is resistant to dissolution in acid media, being stable for passage through the gastric juice, and dissolves rapidly in a neutral to alkaline media, the conditions in the proximal part of the small intestine. In fact, the acid resistance, tested as per US Pharmacopoeia, demonstrated that after minimum 1 hour the total amount of the Lansoprazole remained intact and that upon changing the pH to 6.8, after 30 minutes, tested as per US Pharmacopoeia, practically all the Lansoprazole was dissolved.

### Detailed description of the invention

The new stable oral pharmaceutical preparation containing lansoprazole or a salt thereof as active ingredient, comprises:
a) a nucleus containing an inert core coated with a first layer comprising Lansoprazole or salts thereof, optionally mixed with an alkaline reacting compound, and an inert water soluble polymer,
b) an inert coating disposed on said nucleus, comprising a water soluble polymer; and
c) an enteric outer layer disposed on the previous coating comprising a gastric resistant polymer;
wherein the preparation is characterized in that no anti-tacking agent is present in the first layer or in the inert coating.

In the above definition of the stable oral pharmaceutical preparation the following terms have the meaning indicated.

The term "lansoprazole as active ingredient" refers to lansoprazole or salts thereof, optionally mixed with an alkaline reacting compound.

The term "alkaline reacting excipient or compound" refers to any alkaline compound capable of reacting as a base, neutralizing acids. In other words, an alkaline reacting compound has a substantial chemical effect on this formulation, stabilizing it with respect to acid degradation of lansoprazole. Alkaline reacting excipients are for example magnesium oxide, hydroxide or carbonate, aluminium hydroxide, aluminium, calcium, sodium or potassium carbonate, phosphate or citrate, composite aluminium/magnesium compounds, alkaline aminoacids, N-methyl-D-glucamine, etc.

Excipients that produce neutral or acidic solutions (such as is partially formed by some phases of TiO₂) and substances that are substantially insoluble in diluted acids (such as talc) do not fulfill the criteria required to be alkaline reacting excipients.

In one embodiment the water soluble polymers are for example, cellulose and cellulose derivatives such as sodium carboxymethyl cellulose, ethyl cellulose, hydroxyethyl cellulose, hydroxypropyl cellulose, hydroxypropyl methyl cellulose, methyl cellulose, gelatine, acrylic acid polymers, povidone, povidone K 30. In another embodiment the gastric resistant polymers are selected from co-polymerized methacrylic acid/methacrylic acid methyl ester, carboxymethylethylcellulose (CMEC), hydroxypropylmethylcellulose phthalate (HPMCP), methyl methacrylate-methacrylic acid copolymer (Eudragit L and S; the trade name of Rhom & Haas Co., etc. In another embodiment the plasticizer is triethylcitrate, polyethylene glycol, acetylatedmonoglyceride, triacetin, castor oil, etc. and mixtures thereof.

In a particular embodiment the anti-tacking agent is selected from the group consisting of talc and fumed silica.

In other particular embodiment the water soluble polymer is selected from hydroxipropylmethylcellulose or hydroxypropylcellulose.

A particular embodiment also is that where the gastric resistant polymer is selected from co-polymerized methacrylic acid/ methacrylic acid methyl esters.

In a more particular embodiment the active principle is mixed with an alkaline reacting compound.

In a further particular embodiment the plasticizer is triethylcitrate.

Another aspect of this invention relates to a process for the preparation of a stable oral pharmaceutical preparation containing lansoprazole or salts thereof as active ingredient, which comprises:
a) preparing a nucleus formed by an inert core coated with a first layer comprising lansoprazole or salts thereof, optionally mixed with an alkaline reacting compound, and an inert water soluble polymer,
b) coating said nucleus with an inert layer comprising a water soluble polymer; and
c) coating the previous layer with an enteric coating comprising a gastric resistant polymer such as co-polymerized methacrylic acid/methacrylic acid methyl ester,
wherein the process is characterized in that no anti-tacking agent is added in steps a) or b)

In one embodiment of the process the water soluble polymers are for example, cellulose and cellulose derivatives such as sodium carboxymethyl cellulose, ethyl cellulose, hydroxyethyl cellulose, hydroxypropyl cellulose, hydroxypropyl methyl cellulose or methyl cellulose; gelatin; acrylic acid polymers; and povidone, povidone K 30. In another embodiment of the process the gastric resistant polymers are selected from co-polymerized methacrylic acid/methacrylic acid methyl ester, carboxymethylethylcellulose (CMEC), hydroxypropylmethylcellulose phthalate (HPMCP), methyl methacrylate-methacrylic acid copolymer (Eudragit L and S; the trade name of Rhom & Haas Co., etc.). In another embodiment of the process the plasticizer is triethylcitrate, polyethylene glycol, acetylatedmonoglyceride, triacetin, castor oil, etc. and mixtures thereof.

In a particular embodiment of the process the anti-tacking agent is selected from the group consisting of talc and fumed silica.

In other particular embodiment of the process the water soluble polymer is selected from hydroxipropylmethylcellulose or hydroxypropylcellulose.

A particular embodiment of the process also is that where the gastric resistant polymer is selected from co-polymerized methacrylic acid/ methacrylic acid methyl esters.

In a more particular embodiment, the active principle is mixed with an alkaline reacting compound.

In a particular embodiment the plasticizer is triethylcitrate.

According to a preferred embodiment, in a fluidized bed apparatus, uniform spherical inert cores (composition as per US Pharmacopoeia) are coated with a first layer comprising lansoprazole or salts thereof, and an inert water soluble polymer such as hydroxypropylmethylcellulose or hydroxypropylcellulose. The second layer comprises an inert water soluble polymer such as hydroxypropylmethylcellulose or hydroxypropylcellulose, and a pigment such as titanium dioxide. The third and enteric coating layer comprises an enteric coating polymer such as copolymerized methacrylic acid / methacrylic acid methyl esters, a plasticizer such as triethylcitrate or similar plasticizers, and talc. The layers are applied by conventional fluidized bed coating techniques using aqueous solutions or dispersions.

The active ingredients can be administered in the same dosages and according to the same protocol as the corresponding already marketed commercial dosage forms. For oral administration, the final dosage may take the form of capsules containing the pellets, or pellets compressed into a tablet. The dose as lansoprazole lies within the range of about 1 mg to 100 mg/day, adjusted to individual patients needs and for as long as clinically indicated.

In another aspect the invention is directed to a galenic preparation in the form of capsules, tablets, caplets or losanges containing the stable oral pharmaceutical preparation.

The formulation of the present invention is used for the inhibition of gastric acid secretion, for the treatment or prevention of duodenal ulcer, ventricular ulcer, gastritis, stomach irritation caused by hyperacidity or medicaments, ulcers caused by *Helicobacter pilori,* glaucoma, psoriasis, viral infections, conditions by increasing permeability to chloride ions (brain swelling, renal or heart disease), heartburn symptoms, gastroesophagal reflux disease, dysphasia, airway disorders, non cardiac chest pain, cystic fibrosis, apnoea and snoring, allergies (asthma, urticaria, etc.), postoperative nauseas and vomiting, lower abdominal disorders (IBS, colitis, Crohn's disease), snoring and daytime sleeping, pathological manifestations induced by free radicals, cancer and prophylaxis of cancerous diseases. Therefore, a further aspect of the invention is the use of the pharmaceutical preparation for such uses.

The invention is described in detail in the following examples:

### Example according to the invention

In 3095 g of deionized water, 400 g of lansoprazole and 306 g of hydroxypropylmethylcellulose are dispersed.

2646 g of inert uniform sugar/starch spheres (composition according to US pharmacopoeia) are introduced into a fluidized bed apparatus and the previous obtained dispersion is sprayed on the spheres. After spraying, the spheres are dried before applying the second layer.

In 1909 g of deionized water, 286 g of hydroxypropylmethylcellulose and 38 g of titanium dioxide are dispersed and the resulting aqueous dispersion is sprayed on the spheres obtained in the previous step. After spraying, the spheres are dried before applying the third enteric coating layer.

In 915 g of deionized water, 1588 g of a USP methacrylic acid copolymer (type C aqueous suspension), 72 g of triethylcitrate are dispersed (supension A)

In 616 g of deionized water, 205,5 g of talc is dispersed (suspension B)

Mix suspension A with suspension B and the resulting aqueous dispersion is sprayed on the spheres obtained in the previous step. After applying this final enteric coating layer the spheres (pellets) are dried.

### Example A (comparative)

In 3095 g of deionized water, 400 g of lansoprazole, 96 g of talc and 306 g of hydroxypropylmethylcellulose are dispersed.

2646 g of inert uniform sugar/starch spheres (composition according to US pharmacopoeia) are introduced into a fluidized bed apparatus and the previous obtained dispersion is sprayed on the spheres. After spraying, the spheres are dried before applying the second layer.

In 1909 g of deionized water, 286 g of hydroxypropylmethylcellulose, 38 g of talc and 38 g of titanium dioxide are dispersed and the resulting aqueous dispersion is sprayed on the spheres obtained in the previous step. After spraying, the spheres are dried before applying the third enteric coating layer.

In 915 g of deionized water, 1588 g of a USP methacrylic acid copolymer (type C aqueous suspension), 72 g of triethylcitrate are dispersed (supension A)

In 616 g of deionized water, 72 g of talc is dispersed (suspension B)

Mix suspension A with suspension B and the resulting aqueous dispersion is sprayed on the spheres obtained in the previous step. After applying this final enteric coating layer the spheres (pellets) are dried.

The pellets thus obtained were encapsulated and stored in HDPE containers with tamper-evident ring screw cap closure containing a dessicant capsule and submitted to so called accelerated conditions, that is 40°C and 75% relative humidity during 3 months. At the same time pellets containing lansoprazole obtained according to the comparative example A were stored in identical containers and submitted to the same conditions. The results of the test under accelerated conditions are summarized as follows:

| | Appearance | Lansoprazole purity (Assay by HPLC) | Individual impurities* | Total impurities |
|---|---|---|---|---|
| Example ccording to the invention | Correct | 98.4% | 0.4% | 0.9 % |
| Example A | Correct | 95.0 % | 0.2 % | 0.6 % |

| | | | | |
|---|---|---|---|---|
| * The expression "Individual impurities" refers to the relative amount of the most abundant by-product. | | | | |

It has been demonstrated that the results obtained with both formulations have not significant differences and that the stability profiles are equivalents. Further, the absence of talc in both the active layer and the inert layer did not affect the tackiness of the formulation or the production process.

## Claims

1. A stable oral pharmaceutical preparation containing lansoprazole as active ingredient or salts thereof, which comprises:
a) a nucleus formed by an inert core coated with a first layer comprising lansoprazole or salts thereof and an inert water soluble polymer,
b) an inert coating disposed on said nucleus, comprising a water soluble polymer; and
c) an enteric outer layer disposed on the previous coating comprising a gastric resistant polymer;
wherein the preparation is **characterized in that** no anti-tacking agent is present in the first layer or in the inert coating.

2. A stable oral pharmaceutical preparation according to claim 1, wherein the anti-tacking agent is selected from the group consisting of talc and fumed silica.

3. A stable oral pharmaceutical preparation according to claim 1 or 2, wherein the active principle is mixed with an alkaline reacting compound.

4. A stable oral pharmaceutical preparation according to claims 1 to 3, wherein the water soluble polymer is selected from hydroxypropylmethylcellulose or hydroxypropylcellulose.

5. A stable oral pharmaceutical preparation according to claims 1 to 3, wherein the gastric resistant polymer is selected from co-polymerized methacrylic acid / methacrylic acid methyl esters.

6. A stable oral pharmaceutical preparation according to claims 1 to 3, wherein the plasticizer is triethylcitrate.

7. A process for the preparation of a stable oral pharmaceutical preparation containing lansoprazole as active ingredient or salts thereof, which comprises:
a) preparating a nucleus formed by an inert core coated with a first layer comprising lansoprazole or salts thereof and an inert water soluble polymer,
b) coating said nucleus with an inert layer comprising a water soluble polymer; and
c) coating the previous layer with an enteric coating comprising a gastric resistant polymer such as co-polymerized methacrylic acid/methacrylic acid methyl ester,
wherein the process is **characterized in that** no anti-tacking agent is added in steps a) or b)

8. A process for the preparation of a stable oral pharmaceutical preparation according to claim 7, wherein the anti-tacking agent is selected from the group consisting of talc and fumed silica.

9. A process for the preparation of a stable oral pharmaceutical preparation according to claims 7 or 8, wherein the active principle is mixed with an alkaline reacting compound.

10. A process for the preparation of a stable oral pharmaceutical preparation according to claims 7 to 9 wherein the inert water soluble polymer is selected from hydroxypropylmethylcellulose or hydroxypropylcellulose.

11. A process for the preparation of a stable oral pharmaceutical preparation according to claims 7 to 9 wherein the gastric resistant polymer is selected from co-polymerized methacrylic acid/methacrylic acid methyl ester.

12. A process for the preparation of a stable oral pharmaceutical preparation according to claims 7 to 9 wherein the plasticizer is triethylcitrate.

13. A galenic preparation in the form of capsules, tablets, caplets or losanges containing the stable oral pharmaceutical preparation according to claims 1 to 3.

14. Use of a formulation according to claims 1 or 3 for the preparation of a medicament for the inhibition of gastric acid secretion and for the treatment or prevention of duodenal ulcer, ventricular ulcer, gastritis, stomach irritation caused by hyperacidity or medicaments, ulcers caused by *Helicobacter pilori,* for the treatment or prevention of glaucoma, psoriasis, viral infections, conditions by increasing permeability to chloride ions, heartburn symptoms, gastroesophagal reflux disease, dysphasia, airway disorders, non cardiac chest pain, cystic fibrosis, apnoea and snoring, allergies, postoperative nauseas and vomiting, lower abdominal disorders, snoring and daytime sleeping, pathological manifestations induced by free radicals, cancer or prophylaxis of cancerous diseases.
